# EUROPEAN PATENT SPECIFICATION

(11) **EP 0 335 861 B1**
(45) Date of publication and mention of the grant of the patent: **29.09.1993**
(21) Application number: 87900474.5
(22) Date of filing: 02.12.1986
(51) Int. Cl.: A61B 17/22, A61B 17/32

(54) **CATHETER WITH HIGH SPEED MOVING WORKING HEAD FOR IN-BODY SURGICAL PROCEDURES**
KATHETER MIT HOCHGESCHWINDIGKEITS-ARBEITSKOPF FÜR CHIRURGISCHE EINGRIFFE IM KÖRPERINNERN
CATHETER A TETE DE TRAVAIL MUE A VITESSE ELEVEE POUR INVESTIGATIONS CHIRURGICALES INTRA-CORPORELLES

(30) Priority: 03.10.1986 US 914954; 22.10.1986 US 921973
(43) Date of publication of application: 11.10.1989
(73) Proprietor: KENSEY NASH CORPORATION, Exton, PA 19341 (US)
(72) Inventor: KENSEY, Kenneth, Hinsdale, IL 60521 (US); NASH, John, Downingtown, PA 19355 (US)
(74) Representative: Shaw, Laurence
(86) International application number: US8602616
(87) International publication number: WO8802243

(56) References cited:
- EP-A- 0 191 630

## Description

This invention relates to medical devices and more particularly to flexible, power-driven catheters for intravascular surgery and other in-body surgical procedures.

The known interventional methods for treating atherosclerotic disease involve surgery for bypassing or remoulding obstructive atherosclerotic material. The use of lasers are under investigation for transluminal revascularisation. However, such devices have not found common acceptance in medical practice because of various technical difficulties, the most serious of which being their tendency to perforate arterial tissue.

In US-A-4,445,509 there is disclosed a recanalisation catheter designed specifically for cutting away hard, abnormal deposits, such as atherosclerotic plaque, from the inside of an artery, and while supposedly preserving the soft arterial tissue. That recanalisation catheter includes a sharp edged, multi-fluted, rotary cutting tip mounted at the distal end of the catheter and arranged to be rotated by a flexible drive shaft extending down the centre of the catheter. The rotation of the cutting head is stated as producing a "differential cutting" effect whereupon relatively hard deposits are cut away from relatively soft tissue. Suction ports are provided in the cutting tips to pull the hard particles produced by the cutting action into the catheter for removal at the proximal end thereof so that such particles do not flow distally of the catheter where they could have an adverse effect on the patient's body. EP-A-191630 discloses a similar catheter, but that also has a sharp cutting head.

It has been determined that the use of sharp rotary cutting blades in a revascularisation catheter can have various adverse effects on the arterial tissue, e.g. snagging, cutting or otherwise damaging the tissue of the artery wall.

According to the invention there is provided a catheter for introduction into a lumen in a living being to treat a restriction or a constriction or cause dilation or for disintegration of a stone, the catheter comprising an elongated flexible member having a longitudinal axis, a working head located at the distal end thereof, and elongated flexible drive means therefor extending through the catheter and arranged to cause high speed rotary movement of the working head characterised in that the working head comprises at least one, non-sharp, impacting surface adapted to impact undesirable material or tissue within the lumen or to disintegrate the stone.

Preferably the non-sharp impacting surface is formed as a radiused edge of a cam surface.

Preferably the non-sharp impacting surface has a radius of curvature at least as large as the amplitude of any tissue wave produced by movement of the head with respect of the wall of the lumen.

### Brief Description of the Drawing

Figure 1 is a perspective view, partially in section, showing one embodiment of the catheter of the invention in an artery restricted by occlusive atherosclerotic disease and illustrating the dilation and restriction opening properties of the catheter;
Figure 2 is a side elevational view, partially in section, showing the distal end of the catheter shown in Figure 1;
Figure 3 is a sectional view taken along line 3-3 of Figure 2;
Figure 4 is an end view of the catheter shown in Figure 2;
Figure 5 is a side elevational view of a portion of the distal end of the catheter of Figure 1 and showing the expulsion of fluid therefrom during operation of the catheter;
Figure 6 is a front elevational view of the working head or tip of the catheter shown in Figure 1;
Figure 7 is a side elevational view of the tip shown in Figure 6;
Figure 8 is another side elevational view of the tip shown in Figure 6;
Figure 9 is a side elevational view of a catheter like that shown in Figure 1 located within a bodily lumen, e.g. artery, and illustrating the creation of vortex flow adjacent the working head during the operation of the catheter;
Figure 10 is an enlarged front elevational view of an alternative working head or tip to that shown in Figure 6;
Figure 11 is an enlarged side elevational view of the alternative working tip shown in Figure 10;
Figure 12 is a side elevational view partially in section, showing the distal end of an alternative catheter of the invention;
Figure 13 is a reduced, exploded perspective view of a portion of the distal end of the catheter of Figure 12;
Figure 14 is a reduced side elevational view of the impaction member forming a portion of the catheter of Figure 12; and
Figure 15 is a front elevational view of the impaction member shown in Figure 14.

### DESCRIPTION OF THE INVENTION

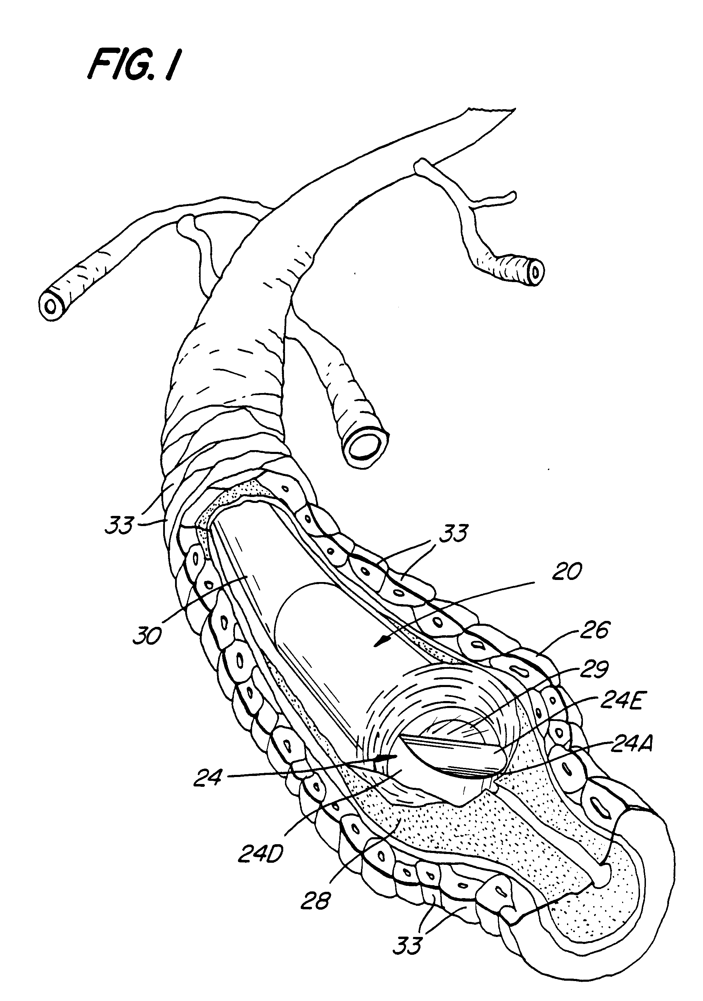

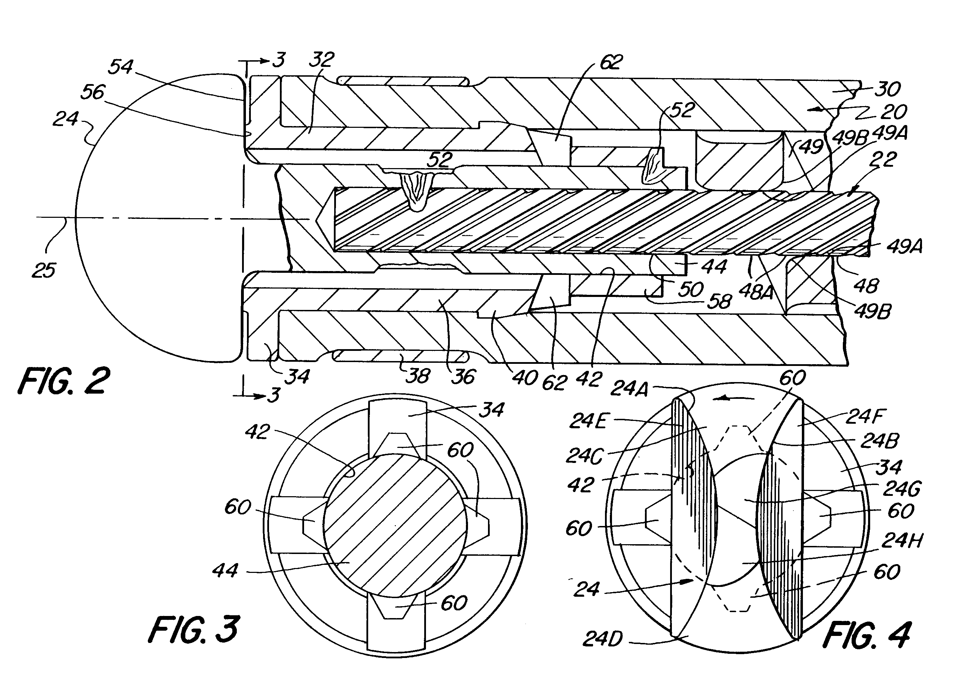

Referring now to the various Figures of the drawing wherein like reference characters refer to like parts, there is shown in Figure 1 the distal end of one embodiment of a catheter 20 for intravascular or other surgical/medical applications, e.g., fallopian tube dilation. The catheter 20 is an elongated flexible member, approximately 180 cm long and of small outside diameter, e.g. 8 French (F) or 2.68 mm, and including a flexible drive means 22 (only a portion of which can be seen in Figure 2) located therein. That drive means will be described later and is particularly suited for in-body surgical applications. Located at the distal end portion of the catheter 20 is a working head 24. The working head can be moved at a high speed with respect to the catheter by the drive means to effect the surgical procedure to be carried out by the catheter. The proximal and of the drive means of the catheter and which is located outside the patient's body is adapted to be connected to a source of rotary power, e.g. an electric motor (not shown). In the preferred embodiment disclosed herein, the drive means 22 effects the rotary movement of the working head 24 under the power provided from the remote power source (motor).

When the catheter 20 is used for treating occlusive atherosclerotic disease, such as opening a restriction in an artery formed by atherosclerotic plaque, the catheter is introduced into the vascular system of the patient, such as through an opening in the femoral artery at a point in the groin. The catheter is then guided through the vascular system of the patient to the site of the restriction so that its working head 24 is located immediately adjacent the restriction. In the illustration in Figure 1, the working head 24 is shown in position in a coronary artery 26 immediately adjacent a restriction 28, e.g. partial occlusion or full occlusion, which is to be opened to the freer flow of blood therethrough.

Such restrictions are formed by the deposit of atherosclerotic plaque or some other material(s), such as wax and/or calcified atheroma, thickened and/or ulcerated intima, etc. The catheter 20 is designed to transluminally recatherise the diseased artery by dilating the stenotic or occluded area (which may or may not be covered by fibrous plaque) and/or selectively removing calcified thrombotic, or fatty tissue unprotected by fibrous plaque while allowing the artery wall to remain intact.

When the catheter is passed transluminally through a diseased artery, the device's working head serves to emulsify occlusive lesions not covered with fibrous plaque by repeatedly impacting the material forming the restriction as the working head is rotated and with minimal risk of puncture or perforation of the contiguous arterial wall.

The rapidly rotating working head removes atherosclerotic tissue by emulsification and differentiates the diseased tissue from the relatively undiseased tissue by using two properties found in normal tissue that is not found in most, if not all, atherosclerotic tissue, namely viscoelasticity and its highly organised fibrous structure.

As can be seen in Figure 1, the fibres 33 of an artery wall 26 run circumferential to the lumen of the artery and generally perpendicular to the impacting surfaces 24A and 24B of the working head where they meet. This perpendicular line between those impacting surfaces and the arterial wall fibres is protective. Thus, the rotating working head does not separate the fibres. Instead, they remain organised in parallel, resisting separation and penetration. The energy absorbed from the rotating working head is distributed through the many fibres, thereby reducing the destructive force applied per fibre. Accordingly, individual fibres are undamaged and the artery wall remains intact.

Atherosclerotic tissue lacks this unified organisation. Thus, when such tissue is engaged by the rotating working head, minute portions of the atherosclerotic tissue must absorb the impacting surfaces' energy alone. Accordingly, a particle of the material breaks off from the adjacent tissue. The operation of the rotary working head creates a vortex flow 31 adjacent the working head which causes the particles broken away by the action of the working head to be repeatedly impacted by the non-sharp, impacting surfaces 24A and 24B, thereby breaking those particles into smaller and smaller particles until they become part of what is effectively a highly emulsified solution.

Referring to Figure 2, the details of the distal end of a preferred embodiment of the catheter 20 will now be described. Catheter 20 basically comprises an elongated, flexible tubular member or jacket 30 which is formed of a suitable material, e.g. plastic, and which has a small outside diameter, e.g. 8F or less. In a preferred embodiment shown herein the outside diameter is approximately 1.7 mm (5F) but it may be as small as, e.g. 2F (.67 mm).

At the distal end of the catheter 20 there is secured a sleeve-like bushing 32. The bushing includes a flanged end face 34 arranged to abut the end of the catheter's jacket 30 and a tubular portion 36. The outside diameter of portion 36 is approximately that of the inside diameter of the catheter's jacket 30 so that it is snugly fit therein. The bushing is held firmly in place by a retaining band 38 which tightly encircles the periphery of the catheter jacket 30 so that plural gripping teeth 40 located about the periphery of the tubular portion 36 dig into the interior surface of the catheter jacket 30 and hold the bushing tightly in place therein. The bushing 32 also includes a through bore 42 (Figures 2 and 3) extending therethrough and aligned with the longitudinal central axis 25 of the catheter.

The working head 24 includes a mounting shank or axle 44 projecting proximally and passing through the bore 42 in the bushing 32. The flexible drive system comprises an elongated drive cable 48 preferably formed of plural elongated wires, e.g. a central wire (not shown) surrounded by six helical wires. The outer surface of the helical wires is swaged or hammered to form a generally large area cylindrical surface 48A (Figure 2). The central wire of the cable 48, may, if desired, be tube. The cable 48 extends down the interior in the catheter's jacket 30 coaxial with axis 25 from a remote, proximally located point (not shown) at which it is connected to a source of power, e.g. an electric motor, to the working head. Thus, the distal end of the cable 48 terminates at and is disposed within a longitudinal extending bore 50 in the axle 44 of the working head 24. The end of the drive cable 48 is secured in place in the bore 50 via a laser weld joint 52. The shape of the working head 24 will be described later. Suffice now to state that it includes a generally planar rear surface 54 which engages the front surface 56 of the bushing flange 34. The working head 24 is prevented from axial movement within the bushing 32 by virtue of a retaining ring 58 mounted on the proximal end of the working head axle 44 contiguous with the proximal end of the bushing. The retaining ring 58 is secured to the proximal end of the working head axle 44 via another laser weld 52.

The drive cable 48 is supported in the central position along axis 25 by means of a spiral bearing 49 (Figure 2) so that the cable can rotate about that axis within the bearing. That bearing member thus comprises a helical or spiral cylindrical coil of wire surrounding the multistrand drive cable 48. The spiral bearing 49 is preferably constructed of a wire having a rectangular cross section. When such a wire is bent into a helix, it forms a central passageway defined by a large area cylindrical surface 49A and with the inside corners of the wire forming each helical convolution being convex at 49B. The bearing 49 extends substantially the entire length of the catheter. The outer diameter of the helical bearing coil is sufficiently great so that its loops just clear the interior surface of the catheter's jacket 30 to hold the bearing securely in place therein. The inside diameter of the central passageway 49A is just slightly greater than the outside diameter of the drive cable 48, that is, the diameter of cylindrical surface 48A, so that the drive cable can freely rotate therein.

The large engaging surfaces 48A and 49A tend to minimise the creation of any wear particles caused by the rotation of cable 48 within bearing 49.

It should also be pointed out that the spiral wire 49 may serve as the drive member (it would be secured to the working head) while the component 48 serves as the bearing.

The drive cable 48 can be rotated at a high rate of speed, e.g. from 10,000 to 200,000 rpm, while the catheter is bent through a small radius of curvature, e.g. .75 inches (1.9 cm), yet maintaining positional neutrality (centering) of the drive cable and without the creation of any standing waves which could result in unwanted vibration to the catheter.

The spacing between the convolutions of the spiral bearing 49, the inner surface of the catheter jacket 30 and the outer surface of the drive cable 48 form a fluid passageway which a fluid (liquid) can flow through the catheter. This liquid is utilised to cool or lubricate the bearing system and also serves to cool the tissue-working head interface.

Moreover, the liquid which is passed down the catheter can, if desired, be oxygenated to eliminate distal ischemia during the restriction opening procedure by the catheter. Also, if desired, nitrates contrast media or other drugs can be added to the liquid as needed during the procedure.

In order to insure that the catheter is sufficiently flexible to negotiate short radii of curvature, while not presenting an undue impediment to the flow of cooling fluid through the fluid passageway in the catheter, the width of the rectangular wire making up the helical coil 48 and the pitch of its loops are appropriately selected. In the embodiment shown and described herein the width (the dimension measured in the direction of axis 25) of the wire making up the helical coil is approximately 0.22 mm for a 5F catheter, while its thickness is approximately 0.16mm and the helix of the angle coils is approximately 30^{o} measured from the axis 25. The outside diameter of the swaged drive cable 48 for a 5F catheter is 0.38mm. This construction optimises bending, flexibility, torsional strength and fluid flow passage. As will be appreciated, the smaller the helix angle the more convolutions of the helix and hence the greater distance through which fluid must flow.

In accordance with the preferred embodiment of the catheter the helical coil bearing 49 is formed of a strong, yet flexible, low friction material, such as heat treated beryllium-copper.

The means for enabling the liquid to exit the catheter at the distal end will now be described with reference to Figures 2,4 and 5.

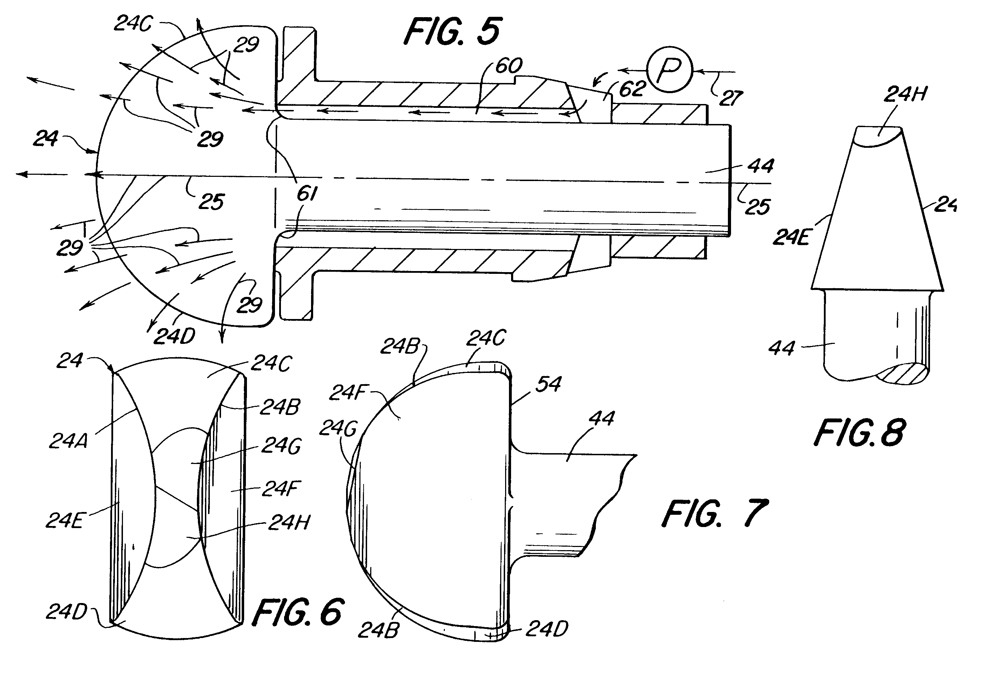

Extending down the central bore 42 of the bearing 32 are four, equidistantly spaced, grooves 60. The distal end of each groove 60 terminates at a fluid exit port 61 located at the distal end flange 34 of the bushing, while the proximal end of each groove 60 terminates in a respective, generally radially extending, relief groove 62. The fluid (liquid) 27 passing down the interior of catheter tube 30 flows under pressure P (Figure 5) into the relief grooves 62, through the associated longitudinal grooves 60 and out through the ports 61 at the end face of the catheter closely adjacent to the longitudinal axis 25.

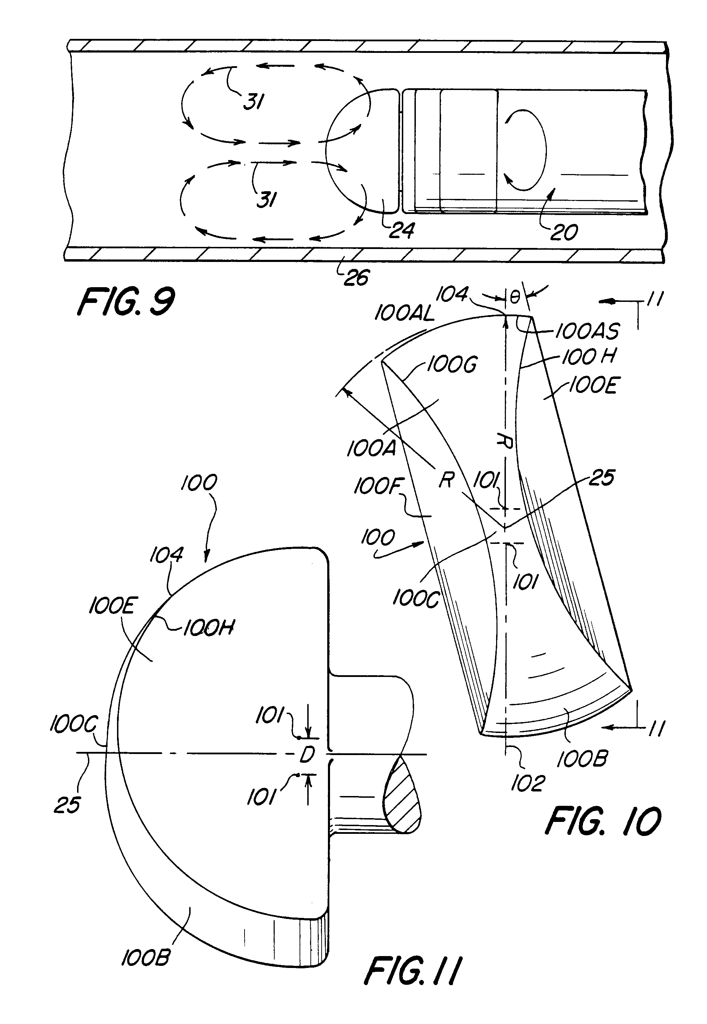

As can be seen in Figures, 2,4,6,7 and 8, the working head 24 basically comprises a convex shaped tip of a generally hemispherical shape and having a pair of generally planar diametrically disposed side faces or relieved surfaces 24C and 24D. Thus, the cam surfaces formed therebetween are sections of the surface of a sphere. The interface of the cam surfaces 24C and 24D with the relieved surfaces 24E and 24F are rounded (radiussed) so that each interface surface is not sharp (although in the scale of the drawings herein it may appear to be a sharp line). As can be seen in Figure 8, the relieved surfaces 24E and 24F taper toward each other in the direction towards the distal end of the working head, with the maximum spacing between the relieved surfaces being approximately the diameter of the working head shaft 44. Thus, the flatted or relieved surfaces are at a negative rake angle to the cam surfaces. Further details of the working head will be described later.

As can be seen in Figures 4 and 5, by virtue of the shape of the working head the fluid exit ports 61 are uncovered by the relieved surfaces 24E and 24F to enable fluid 27 to exit the ports 61. As the working head rotates, its relieved surfaces sequentially cover and uncover ports 61. This action breaks up the fluid streams 27 exiting from those ports into slugs 29.

The fluid velocity is determined by the pressure at the point P in Figure 5. For catheters of an 8F (French) size, and whose working head is of 1.25 mm radius a pressure of approximately 2.07·10³ N/m² (30 pounds per square inch) is deemed sufficient to ensure that some liquid streams flow axially along axis 25 so that the exiting liquid is distributed in a generally hemispherical pattern about the working head. For catheters of 5F (French), a pressure of 6.89·10⁵ N/m² (100 PSI) is sufficient. Accordingly, with sufficient fluid pressure applied some of the liquid streams reach the end of the tip contiguous with the longitudinal central axis 25 while other streams are cut off and accelerated at an acute angle thereto and still further streams are cut off and accelerated almost radially. Accordingly, the fluid exiting from the ports is distributed almost hemispherically around the tip without the need for a central hole therein.

In order to prevent heat induced injury to the artery, sufficient liquid should be expelled into the restriction at the working head. It has been found that 30 ccs per minute is suitable for an 8F (French) instrument while 20 ccs per minute is suitable for a 5F instrument.

Many of the liquid slugs 29 have some radial component and develop tremendous momentum as they are flung outwards toward the artery wall. The momentum of the slugs is transferred to the artery wall, thereby forcing the wall laterally outward in all radial directions to dilate it.

Tests have shown that the radial pressure developed by the rotating working head is substantial and can raise local static pressure immediately adjacent the working head by approximately 1.33·10⁴ to 2.66·10⁴ N/m² (100 to 200 millimetres of Hg). This increased pressure on the artery wall contiguous with the rotating working head is not due solely to the impact of the fluid slugs thereon, but is also due to the recirculation of the fluid surrounding the working head. The rotation of the working head about axis 25 produces a powerful, toroidal shaped vortex 31 contiguous with the working head as shown in Figure 9. The vortex 31 also has the effect of recirculating any particles broken off from the restriction by the impact of the rotating working head. Thus particles that break away are carried back into the rotating working head where they are progressively reduced in size. This produces particles which are safe to flow distally. In this connection, it has been determined that in a typical operation 95% of the particles created during the impacting or emulsification process have a surface area smaller than that of a red blood cell.

The impacting surfaces are shown at 24A and 24B (see Figure 6). They are the interfacial areas at which the spherical section cam surfaces 24C and 24D meet the relieved surfaces 24E and 24F, are of sufficiently large radius to ensure that no damage to the healthy tissue of the artery occurs when those surfaces impact arterial tissue. In this regard, the visoleastic nature of healthy tissue as well as diseased soft tissue is such that such soft materials can be stretched and negotiated by the rotating working head if its impacting surfaces are of sufficiently large radius that they allow the arterial tissue (in the form of a wave of tissue) to flow smoothly thereunder. Moreover the cam surface passing frequency, that is, the velocity of the cam surface coupled with the length of the cam surface should be large enough that the tissue cannot recover substantially before the next impacting suface arrives. This allows quite aggressive instrument feed rates without puncture. In this connection, it is suggested that the velocity of the impacting surfaces 24A and 24B at their maximum distance from axis 25 be in the range from 100 to 2,000 centimeters per second. This speed range ensures that at the low end the impacting surfaces of the rotating working head always describe a fine helix in the artery even at high feed rates of the order of ten centimetres per second. This makes use of the protective nature of the tip travel along the axis of the circumferential arterial wall fibres. The radius of the impacting surfaces 24A and 24B should also not be too large.

In order not to compromise the above described vortex action, it is necessary that the working head impacting surface radius not be too large to compromise the particle reduction action. For tips having an impacting surface radius of 0.05 to 0.07 mm the progressive particle reduction action operates at tip rotational speeds of 30,000 to 90,000 rpm.

As just discussed, injury to soft tissue is controlled by the impacting surface radius and its passing velocity and to a lesser degree by its and the contiguous cam surface's clearance. However, hard tissue seems to be dramatically affected by clearance, with the smaller clearance, the less chance of injuring or perforating the arterial tissue. Directional protection control can also be achieved by varying the clearance of the working head's impacting surface radius. Hence, as can be seen in Figures 6,7 and 8, the portion of the working tip cam surfaced 24C and 24D contiguous with the rotational axis 25 and 45 and is relieved by the formation of two diametrically opposed planar sections 24G and 24H. Thus, the radiused impacting surfaces at the interface of the cam surfaces and the planar relieved surfaces have approximately zero degree clearance while the radiused impacting surfaces at the interface of cam surfaces and the relieved surfaces form a ten degree clearance. Accordingly, the working head 24 of the subject invention has zero clearance at large radial distances from the rotational axis 25 and ten degree clearance at small radial distances. This feature compensates, for the lower velocity of the impacting surfaces at smaller radial distances. Accordingly, in accordance with the subject invention, working heads can be produced to provide very small clearance at portions of the working head moving at high speed with respect to the material to be removed while providing some larger clearance at portions of the tip moving at lower speeds with respect to that material.

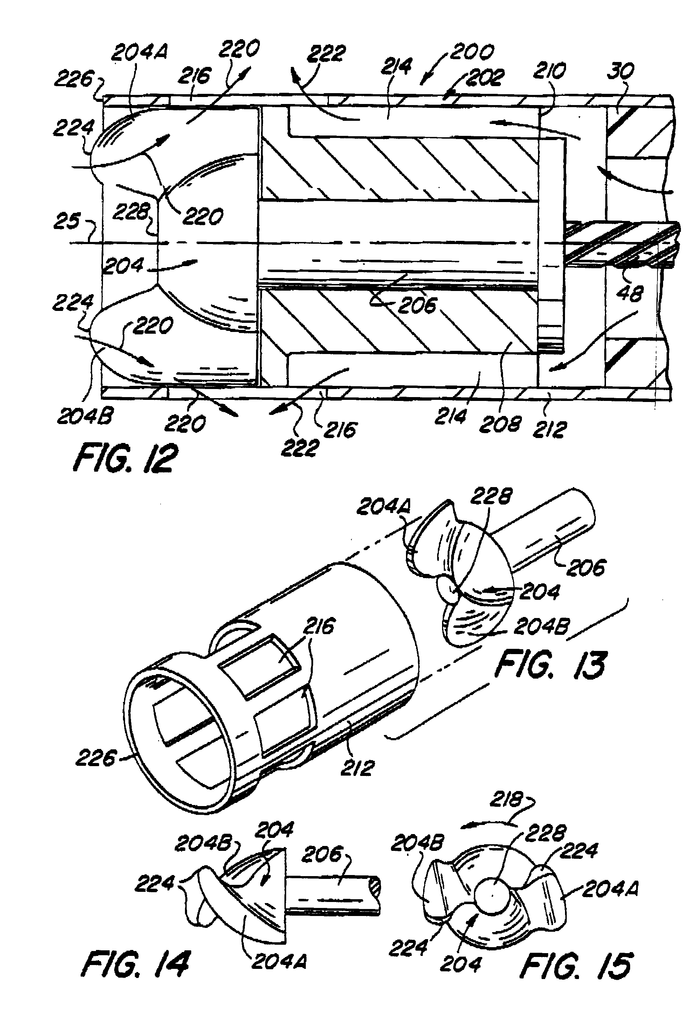

In order to produce an even more gentle action on the arterial tissue wave created by the rotating cammed working head, it can be constructed as shown in Figures 10 and 11 and denoted by the reference numeral 100. Thus, in the working head 100 the convex cam surface is not of a constant radius of curvature. Working head 100 includes two quadraspherical section cam surfaces 100A and 100B, each of which has the same radius of curvature. The centers of generation of the quadraspheres are denoted by the reference numeral 101 and are spaced (offset) from each other by a distance D (Fig.11). Accordingly, the surfaces 100A and 100B are separated from each other by an intermediate surface 100C whose width is D. As can be seen in Figure 11, the surface 100C is tangential to the ends of the opposed quadraspherical surfaces 100A and 100B and is linear between the ends of those surfaces when viewed in the direction of lines 11-11 in Figure 10 but circular and of the same radius as surfaces 100A and 100B measured around an axis 102 (Figure 10). The plane (the working head bisecting plane) in which the axes 25 and 102 lie includes the two centres of generation 101 and bisects the working head 100 into two halves. The flatted or relieved surfaces 100D and 100E are similar to relieved surfaces 24E and 24F of working head 24. However, as can be seen in Figure 10, the relieved surfaces 100F and 100E are oriented at an angle ϑ with respect to the working head bisecting plane. Thus, the working head 100 is bisected into two symmetric portions by the working head bisecting plane. This construction results in the creation of a long ramp cam surface 100AL between the leading radiused impacting surface 100G and the highest point 104. The ramp can be appreciated by viewing the difference between the path of maximum radius R generated by the rotation of head 24 and the surface 100AL while creating a short ramp surface 100AS between point 104 and the trailing radiused impacting surface 100H. By virtue of the relatively long ramp cam surface 100AL leading to the point of maximum cam surface radius a gentle cam action results when the surface 100AL makes contact with the material forming the restriction to result in lower acceleration (less aggression) applied to the particles produced by that impact. In alternative embodiments of the working head 100 the head bisecting plane can be oriented so that the angle ϑ is between zero degrees and any maximum angle. If the head bisecting plane is parallel to the relieved surfaces 100E and 100F so that the angle ϑ is zero degrees then the leading and trailing cam surfaces 100AL and 100AS will be the same length.

Thus by adjusting the orientation of the head bisecting angle, one can adjust the degree of aggression of the working head.

Other shaped working heads can be constructed in accordance with this invention. Thus, the cam surfaces need not be portions of a spherical surface, but can be ovoidal, conical or any other suitable shape. Moreover, the relieved surfaces need not be planar, but can be arcuate, multiplanar (portions in different planes), etc. Further still, the impacting surfaces need not be of a constant radius so long as they are sufficiently rounded or arcuate to be substantially equal to or larger than the tissue wave to be created by the rotation of the working head.

The mechanical and fluid forces applied by the working head allow the catheter to track the point of least resistance in total occlusions. In this regard, the working head finds the area of least resistance by dissecting the tissue with fluid pressure as it moves forward. From observation, the point of least resistance is always in the lumen of the previously patent artery. It is therefore relatively easy and safe to open totally obstructed tortuous arteries with the subject catheter. In this connection, the working head finds the area of least resistance and serves to guide the catheter and not vice versa.

Referring now to Figures 12-15, the details of the catheter 200 having particular utility for effecting the disintegration of the bodily stones or for accomplishing an in-situ valvulectomy will now be described.

The catheter 200 in many respects is identical in construction to the catheters 20 described heretofore. Hence the same reference numerals will be used for the common components. In particular the drive means and bearing means for the drive means can be the same as in either catheter 20, whereas the working head 202 is considerably different. In this regard the working head basically comprises a propeller-like impacting member 204 having a pair of blades 204A and 204B. The member 204 is threadedly engaged (not shown) onto a central mounting shaft 206 fixedly secured within an inner bushing 208. The inner bushing is disposed within an outer bushing 210. The outer bushing is fixedly secured within a tubular shroud 212. The distal end of the drive cable 48 is secured within the shaft 206, whereupon the shaft and the inner bushing to which it is connected are arranged to rotate about central axis 25 under power from a remote motor (not shown). A plurality of slots 214 are provided along the outer bushing between it and the shroud 212. These slots serve as the fluid passageways (to be described later).

The shroud 212 serves to protect the surrounding bodily tissue(s), e.g. duct walls, from damage caused by the rotary motion of the blades 204A and 204B during the stone disintegration procedure. To that end the shroud is fixedly secured onto the distal end of the catheter jacket 30 by a retaining band (not shown) like that described with reference to Figure 2. As can be seen clearly in Figures 12 and 13, the shroud includes a plurality of generally rectangularly shaped windows 216 disposed about the periphery thereof adjacent its distal end (for reasons to be described later).

Each blade 204A and 204B of the impacting member 204 is curved and has a lead angle (Figures 14 and 15) arranged so that upon rotation of the member about the longitudinal axis 25 of the catheter in the direction of arrow 218 (Figure 15) a toroidal vortex fluid flow path is induced distally of the working head. This vortex flow is denoted by the arrows 220 and is in the direction towards the working head. This flow serves to draw the stone or venous valves to be acted upon by the catheter 200 into contact with the blades 204A and 204B. This in-drawing feature is of significant importance in applications wherein the stone or tissue to be destroyed is loose and not restrained by some body tissue, in order to ensure that the working does engage the stone or tissue to effect its destruction.

As can be seen in Figure 12, the slots 214 are located between the bushing 208 and the inner surface of the shroud 212 and serve as passageways enabling the fluid (liquid) flowing through the interior of the catheter (and serving to cool and lubricate the moving components thereof as described with reference to the embodiments of Figure 1) to flow through the shroud's windows 216 out of the working head in the direction of arrows 222. The induced fluid flow distally of the head caused by the rotating blades also flows into the head and through the windows 216 (as shown by the arrows 220). These combined flows serve to push the duct walls or body tissue surrounding the stone away from the shroud 212, thereby aiding the shroud in protecting the patient from injury caused by the rotating blades.

As can be seen in Figures 14,15 each blade tip is slightly rounded at 224 and projects a short distance, e.g. 0.13 to 0.25 mm, beyond the distal end 226 of the shroud 212. The rounded surfaces 224 serve as impact hammers to pulverize the stone upon contact therewith. In order to maximise the impact force the member 204 is relieved centrally at 228 so that the impaction surfaces 224 are located a substantial radial distance from the central axis 25 about which the member 204 rotates.

Operation of the catheter 200 to disintegrate a stone, such as a bile duct stone (not shown) is as follows. The catheter is introduced into an appropriate portion. e.g. the cystic duct, and then guided into the bile duct in which the stone is located and then until its working head is just proximately of the position of the stone. When the drive means is operated it causes the head 204 to rotate at a high rate of speed, e.g. 100,000 RPM or greater, thereby creating the vortex flow 220 which draws the stone into contact with the advancing head. The rapid rotation of the head 204 causes the impaction surfaces 224 to rapidly hammer away at the stone. This action pulverizes the stone, thereby resulting in its disintegration into particles of sufficiently small size as to be flushed from the duct by bodily fluids. The catheter is then withdrawn.

Operation of the catheter 200 to effect the in-situ destruction of valves in a section of vein (not shown) is accomplished in a similar manner. Thus the advancement of the catheter distally, coupled with the vortex flow at the distal end thereof, draws the fragile valve elements of the vein into contact with the rotating blades 204A and 204B, whereupon the valve elements are destroyed or otherwise rendered inoperative. It should be noted that if the clearance between the blades and windows 216 is kept sufficiently small a shearing force results therebetween to aid in destroying the fragile valve elements.

It must be pointed out at this juncture that the other catheters, such as the heretofore described catheter 20, can be used to effect stone disintegration or in situ valvulectomy in lieu of the catheter 200. Thus, for some applications the working heads may be constructed like heads 24 and 100, with no shroud necessary.

## Claims

1. A catheter (20,100,200) for introduction into a lumen in a living being to treat a restriction or a constriction or cause dilation or to disintegrate a stone; the catheter (20,100,200) comprising an elongated flexible member having a longitudinal axis (25,102), a working head (24,100,202) located at the distal end thereof, and elongated flexible drive means (22) therefor extending through the catheter and arranged to cause high speed rotary movement of the working head (24,100,202) characterised in that the working head (24,100,202) comprises at least one, non-sharp, impacting surface (24A,24B,100A,100B,204A,204B), adapted to impact undesirable material or tissue within the lumen or to disintegrate the stone.

2. A catheter according to Claim 1 characterised in that the non-sharp impacting surface (24A,24B) is formed as a radiused edge of a cam surface (24C,24D).

3. A catheter according to Claim 1 or 2 characterised in that two cam surfaces (24C and 24D) are present and located between relieved surfaces (24E and 24F).

4. A catheter according to Claim 1, 2 or 3 characterised in that the non-sharp impacting surface (24A, 24B, 100A, 100B, 204A, 204B) has a radius of curvature at least as large as the amplitude of any tissue wave produced by movement of the head (24, 100, 202) with respect to the wall (26) of the lumen.

5. A catheter according to Claim 2, 3 or 4 characterised in that the height and profile of the cam surfaces (24C,24D) and the radiused impacting surfaces (24A, 24B) are selected so that the healthy wall of the lumen (26) will deform viscoelastically under applied load while non-viscoelastic material will be impacted.

6. A catheter according to Claim 5 characterised in that the impacting surfaces (24A, 24B) have a radius in the range of 0.025 mm (0.001 inch) to 0.127 mm (0.005 inch).

7. A catheter according to any preceding Claim characterised in that the head (24,100,202) is arranged to be rotated about the longitudinal axis (25,102) of the catheter.

8. A catheter according to Claim 7 characterised in that the working head (24,100,202) is arranged to be rotated about the longitudinal axis (25) of the catheter at about 10,000 to 200,000 rpm.

9. A catheter according to any preceding Claim including a jacket (30) and housing a drive axle (48) with a passageway for a stream of fluid (27) to pass to the head (24) in between, the fluid emerging adjacent the head (24) via exit ports (61) characterised in that the ports (61) are located adjacent the longitudinal axis (25) of the catheter so that the fluid (27) is broken into substreams (29) which are directed radially outwardly as the head (24) is rotated.

10. A catheter according to Claim 9 characterised in that the head (24) includes surface portions (24E,24F) arranged to expose or cover the exit ports (61) as the head (24) is rotated.

11. A catheter according to Claim 9 or 10 characterised in that the fluid (27) is supplied under a pressure (P) selected so that the exiting substreams (29) present a hemispherical pattern about the head (24).

12. A catheter according to any preceding Claim characterised in that the head (24) is rotated at sufficient speed to form a fluid path (31) in advance of the head (24) whereby particles of an impacted restriction (28) are drawn into the head (24) for repeated treatment.

13. A catheter according to Claim 12 characterised in that the fluid path (31) is a toroidal vortex.

14. A catheter (100) according to any preceding Claim characterised by a head (100) having a convex cam surface of non constant radius curvature bisected into two symmetrical portions by a bisecting plane to define a long ramp cam surface (100AL) and a short ramp surface (100AS) to cause a gentle cam action on a tissue wave generated in use of the head.

15. A catheter (200) according to Claim 1, 7, 8, 12, 13 characterised in that the head (204) has blades (204A, 204B) at the distal end of a rotary shaft (206) disposed and projecting slightly forward of a shroud (212) having windows (216) about the blades (204A, 204B), rotation of the shaft (206) being arranged to create a vortex (220,222) to draw fluid past the blades (204A,204B) and out of the windows (216).

16. A catheter according to Claim 15 characterised in that the blades (204A,204B) have rounded impact surfaces (224).

17. A catheter (200) according to any one of Claims 1,4,7,8,9,10,11,12,13,15 and 16 characterised in that the working head comprises a propeller-like impact member (204) having rounded impacting surfaces (224) spaced from the longitudinal axis (25) and in that a shroud (212) is radially spaced from and extends about the impacting member (204) so that the rounded impacting surfaces (224) project a short distance beyond the shroud (212) which protects the rounded impacting surfaces (224) from damaging surrounding body tissues.

18. A catheter according to Claim 17 or 18 characterised in that the shroud (212) has windows (216) about the working head (202) and rotation of a shaft (206) is arranged to create a vortex (220,222) to draw fluid past the impacting surfaces (224) and out through the windows (216).

19. A catheter according to any preceding Claim characterised in that the drive assembly (22) comprises an elongated drive cable (48) arranged to rotate within a spiral bearing (49) extending along the catheter.

20. A catheter according to Claim 20 characterised in that the spiral bearing (49) is formed of a wire having a generally rectangular cross sectional shape.

21. A catheter according to Claim 20 or 21 characterised in that the cable (48) and the bearing (49) have large engaging surfaces (48A,49A) respectively to reduce wear.

## Patentansprüche

1. Katheter (20, 100, 200) zur Einführung in ein Lumen in einem lebenden Wesen, um eine Verstopfung oder eine Einschnürung zu behandeln, oder um eine Erweiterung zu verursachen, oder um einen Stein zu zertrümmern; wobei der Katheter (20, 100, 200) ein langgestrecktes biegsames Teil umfaßt, das eine Längsachse (25, 102) aufweist, einen Arbeitskopf (24, 100 202), der an seinem distalen Ende angeordnet ist und ein langgestrecktes biegsames Antriebsmittel (22) dafür, das sich durch den Katheter erstreckt und dazu ausgelegt ist, eine Drehbewegung des Arbeitskopfes (24, 100, 202) mit hoher Drehzahl zu verursachen,
dadurch **gekennzeichnet,**
daß der Arbeitskopf (24, 100, 202) wenigstens eine nicht scharfe Stoßoberfläche (24A, 24B, 100A, 100B, 204A, 204B) umfaßt, die dazu ausgelegt ist, unerwünschtes Material oder Gewebe innerhalb des Lumens mit Stoß zu beaufschlagen oder den Stein zu zertrümmern.

2. Katheter nach Anspruch 1,
dadurch **gekennzeichnet,**
daß die nicht scharfe Stoßoberfläche (24A, 24B) als verrundete Kante einer Nockenoberfläche (24C, 24D) ausgebildet ist.

3. Katheter nach Anspruch 1 oder 2,
dadurch **gekennzeichnet,**
daß zwei Nockenoberflächen (24C und 24D) vorhanden und zwischen ausgesparten Oberflächen (24E und 24F) angeordnet sind.

4. Katheter nach Anspruch 1, 2 oder 3,
dadurch **gekennzeichnet,**
daß die nicht scharfe Stoßoberfläche (24A, 24B, 100A, 100B, 204A, 204B) einen Krümmungsradius aufweist, der wenigstens so groß ist wie die Amplitude einer beliebigen Gewebewelle, die durch eine Bewegung des Kopfs (24, 100, 202) mit Bezug auf die Wand (26) des Lumens erzeugt ist.

5. Katheter nach Anspruch 2, 3 oder 4,
dadurch **gekennzeichnet,**
daß die Höhe und das Profil der Nockenoberflächen (24C, 24D) und die verrundeten Stoßoberflächen (24A, 24B) so ausgewählt sind, daß sich die gesunde Wand des Lumens (26) unter angelegter Last viscoelastisch deformiert, während nicht-viscoelastisches Material gestoßen wird.

6. Katheter nach Anspruch 5,
dadurch **gekennzeichnet,**
daß die Stoßoberflächen (24A, 24B) einen Radius im Bereich von 0,025 mm (0,001 Zoll) bis 0,127 mm (0,005 Zoll) aufweisen.

7. Katheter nach einem der vorangehenden Ansprüche,
dadurch **gekennzeichnet,**
daß der Kopf (24, 100, 202) dazu ausgelegt ist, um die Längsachse (25, 102) des Katheters gedreht zu werden.

8. Katheter nach Anspruch 7,
dadurch **gekennzeichnet,**
daß der Arbeitskopf (24, 100, 202) dazu ausgelegt ist, um die Längsachse (25) des Katheters mit etwa 10.000 bis 200.000 UpM gedreht zu werden.

9. Katheter nach einem der vorangehenden Ansprüche,
der eine Hülse (30) umfaßt und eine Antriebswelle (48) mit einem Durchgang für einen Fluidstrom (27) zum Kopf (24) im Zwischenraum enthält, wobei das Fluid benachbart zum Kopf (24) über Ausgangsöffnungen (61) austritt,
dadurch **gekennzeichnet,**
daß die Öffnungen (61) benachbart zur Längsachse (25) des Katheters so angeordnet sind, daß das Fluid (27) in Unterströme (29) aufgebrochen wird, die radial nach außen gerichtet sind, wenn der Kopf (24) drehangetrieben ist.

10. Kathether nach Anspruch 9,
dadurch **gekennzeichnet,**
daß der Kopf (24) Oberflächenabschnitte (24E, 24F) umfaßt, die dazu ausgelegt sind, die Ausgangsöffnungen (61) freizugeben oder zu bedecken, wenn der Kopf (24) drehangetrieben ist.

11. Katheter nach Anspruch 9 oder 10,
dadurch **gekennzeichnet,**
daß das Fluid (27) unter Druck (P) zugeführt wird, der so ausgewählt ist, daß die austretenden Unterströme (29) ein halbkugelförmiges Muster um den Kopf (24) herum darstellen.

12. Katheter nach einem der vorausgehenden Ansprüche,
dadurch **gekennzeichnet,**
daß der Kopf (24) mit einer ausreichenden Drehzahl drehangetrieben wird, um einen Fluid-Pfad vor dem Kopf (24) zu bilden, wodurch Teilchen einer gestoßenen Verstopfung (28) in den Kopf (24) für eine wiederholte Behandlung hineingezogen werden.

13. Katheter nach Anspruch 12,
dadurch **gekennzeichnet,**
daß der Fluid-Pfad (31) ein ringförmiger Wirbel ist.

14. Katheter (100) nach einem der vorangehenden Ansprüche,
**gekennzeichnet** durch
einen Kopf (100), der eine konvexe Nockenoberfläche mit einer Krümmung nicht konstantem Radius aufweist, die durch eine halbierende Ebene in zwei symmetrische Abschnitte halbiert ist, um eine lange Rampennockenoberlfäche (100AL) festzulegen sowie eine kurze Rampenoberfläche (100AS), um eine sanfte Nockenwirkung auf eine Gewebewelle zu verursachen, die bei Verwendung des Kopfes erzeugt ist.

15. Katheter (200) nach Anspruch 1, 7, 8, 12, 13,
dadurch **gekennzeichnet,**
daß der Kopf (204) Schaufeln (204A, 204B) an dem distalen Ende einer Drehwelle (206) aufweist, die angeordnet sind und leicht nach vorne vorstehen über eine Abdeckung (212), die Fenster (216) über den Schaufeln (204A, 204B) aufweist, wobei die Drehung der Welle (206) so ausgelegt ist, daß ein Wirbel (220, 222) erzeugt wird, um Fluid hinter die Schaufeln (204A, 204B) sowie aus den Fenstern (216) hinaus zu ziehen.

16. Katheter nach Anspruch 15,
dadurch **gekennzeichnet,**
daß die Schaufeln (204A, 204B) verrundete Stoßoberflächen (224) aufweisen.

17. Katheter (200) nach einem der Ansprüche 1, 4, 7, 8, 9, 10, 11, 12, 13, 15 und 16,
dadurch **gekennzeichnet,**
daß der Arbeitskopf ein propellerartiges Stoßteil (204) umfaßt, das verrundete Stoßoberflächen aufweist, die von der Längsachse (25) beabstandet sind, und daß eine Hülle (212) radial beabstandet ist von und sich herum erstreckt um das Stoßteil (204) so, daß die verrundeten Stoßoberflächen (224) um ein kurzes Stück über die Hülle (212) vorstehen, die die verrundeten Stoßoberflächen (212) davon abhält, umgebendes Körpergewebe zu beschädigen.

18. Katheter nach Anspruch 17 oder 18,
dadurch **gekennzeichnet,**
daß die Hülle (212) Fenster (216) über dem Arbeitskopf (202) aufweist, und das Drehen einer Welle (206) so ausgelegt ist, daß ein Wirbel (220, 222) erzeugt wird, um Fluid hinter die Stoßoberflächen (224) sowie durch die Fenster (216) hinaus zu ziehen.

19. Katheter nach einem der vorausgehenden Ansprüche,
dadurch **gekennzeichnet,**
daß die Anstriebsanordnung (22) ein längsgestrecktes Antriebskabel (48) umfaßt, das dazu ausgelegt ist, sich innerhalb eines Spirallagers (49) zu drehen, das sich entlang des Katheters erstreckt.

20. Katheter nach Anspruch 19, dadurch gekennzeichnet,
dadurch **gekennzeichnet,**
daß das Spirallager (49) aus einem Draht gebildet ist, der eine allgemein rechteckige Querschnittsform aufweist.

21. Katheter nach Anspruch 20 oder 21,
dadurch **gekennzeichnet,**
daß das Kabel (48) und das Lager (49) große Eingriffsoberflächen (48A, 49A) aufweisen, um Abnutzung zu vermindern.

## Revendications

1. Cathéter (20, 100, 200) destiné à être introduit dans un canal d'un être vivant pour traiter une restriction ou une constriction, pour provoquer une dilatation ou pour désintégrer un calcul; le cathéter (20, 100, 200) comportant un organe flexible allongé qui possède un axe longitudinal (25, 102), une tête de travail (24, 100, 202) située au niveau de son extrémité distale, et, pour celle-ci, des moyens d'entraînement flexibles allongés (22) qui s'étendent à travers le cathéter et sont conçus pour provoquer un mouvement de rotation à grande vitesse de la tête de travail (24, 100, 202), caractérisé en ce que la tête de travail (24, 100, 202) comporte au moins une surface de percussion non tranchante (24A, 24B, 100A, 100B, 204A, 204B) adaptée pour percuter une substance ou un tissu indésirable présent(e) à l'intérieur du canal ou pour désintégrer le calcul.

2. Cathéter selon la revendication 1, caractérisé en ce que la surface de percussion non tranchante (24A, 24B) se présente sous la forme d'un bord arrondi d'une surface formant came (24C, 24D).

3. Cathéter selon la revendication 1 ou 2, caractérisé en ce qu'il existe deux surfaces formant came (24C et 24D) situées entre des surfaces dépouillées (24E et 24F).

4. Cathéter selon la revendication 1, 2 ou 3, caractérisé en ce que la surface de percussion non tranchante (24A, 24B, 100A, 100B, 204A, 204B) a un rayon de courbure au moins aussi important que l'amplitude d'une quelconque ondulation de tissu produite par le déplacement de la tête (24, 100, 202) par rapport à la paroi (26) du canal.

5. Cathéter selon la revendication 2, 3 ou 4, caractérisé en ce que la hauteur et le profil des surfaces formant came (24C, 24D) et des surfaces de percussion arrondies (24A, 24B) sont sélectionnés de façon que la paroi saine du canal (26) se déforme viscoélastiquement sous l'effet de la charge appliquée, pendant que la substance non viscoélastique sera percutée.

6. Cathéter selon la revendication 5, caractérisé en ce que les surfaces de percussion (24A, 24B) ont un rayon qui se situe dans la plage de 0,025 mm (0,001 pouce) à 0,127 mm (0,005 pouce).

7. Cathéter selon l'une quelconque des revendications précédentes, caractérisé en ce que la tête (24, 100, 202) est conçue pour être animée d'un mouvement de rotation autour de l'axe longitudinal (25, 102) du cathéter.

8. Cathéter selon la revendication 7, caractérisé en ce que la tête de travail (24, 100, 202) est conçue pour être animée d'un mouvement de rotation autour de l'axe longitudinal (25) du cathéter à environ 10 000 à 200 000 tr/min.

9. Cathéter selon l'une quelconque des revendications précédentes, comportant une gaine (30) et logeant un axe d'entraînement (48), un passage étant ménagé entre eux pour permettre l'écoulement d'un jet de fluide (27) en direction de la tête (24), le fluide débouchant à proximité de la tête (24) par des orifices de sortie (61), caractérisé en ce que les orifices (61) sont situés à proximité de l'axe longitudinal (25) du cathéter de façon que le fluide (27) soit décomposé en jets secondaires (29) dirigés radialement vers l'extérieur, lorsque la tête (24) est animée d'un mouvement de rotation.

10. Cathéter selon la revendication 9, caractérisé en ce que la tête (24) comporte des parties de surface (24E, 24F) disposées pour exposer ou recouvrir les orifices de sortie (61), lorsque la tête (24) est animée d'un mouvement de rotation.

11. Cathéter selon la revendication 9 ou 10, caractérisé en ce que le fluide (27) est délivré sous une pression (P) sélectionnée de façon que les jets secondaires (29) qui sortent, présentent une configuration hémisphérique autour de la tête (24).

12. Cathéter selon l'une quelconque des revendications précédentes, caractérisé en ce que la tête (24) est animée d'un mouvement de rotation à une vitesse suffisante pour définir une trajectoire de fluide (31) en avant de la tête (24), afin qu'ainsi des particules d'une restriction (28) percutée soient entraînées dans la tête (24) en vue d'un traitement répété.

13. Cathéter selon la revendication 12, caractérisé en ce que la trajectoire de fluide (31) est un vortex toroïdal.

14. Cathéter (100) selon l'une quelconque des revendications précédentes, caractérisé par une tête (100) qui présente une surface formant came convexe ayant une courbure de rayon non constant, divisée en deux parties égales symétriques par un plan bissecteur pour définir une surface formant came inclinée longue (100AL) et une surface inclinée courte (100AS), afin de provoquer un effet de came modéré sur une ondulation de tissu produite lors de l'utilisation de la tête.

15. Cathéter (200) selon la revendication 1, 7, 8, 12, 13, caractérisé en ce que la tête (204) possède, au niveau de l'extrémité distale d'un arbre de rotation (206), des lames (204A, 204B) disposées pour faire saillie légèrement vers l'avant d'une enveloppe (212) pourvue de fenêtres (216) autour des lames (204A, 204B), une rotation de l'arbre (206) étant conçue pour créer un vortex (220, 222) afin de diriger un fluide devant les lames (204A, 204B) et hors des fenêtres (216).

16. Cathéter selon la revendication 15, caractérisé en ce que les lames (204A, 204B) présentent des surfaces de percussion arrondies (224).

17. Cathéter (200) selon l'une quelconque des revendications 1, 4, 7, 8, 9, 10, 11, 12, 13, 15 et 16, caractérisé en ce que la tête de travail comporte un organe de percussion semblable à une hélice (204) qui présente des surfaces de percussion arrondies (224) espacées de l'axe longitudinal (25), et en ce qu'une enveloppe (212) est espacée radialement de l'organe de percussion (204) et s'étend autour de celui-ci de façon que les surfaces de percussion arrondies (224) fassent saillie sur une courte distance au-delà de l'enveloppe (212) qui protège des tissus du corps environnants contre une détérioration par les surfaces de percussion arrondies (224).

18. Cathéter selon la revendication 17 ou 18, caractérisé en ce que l'enveloppe (212) possède des fenêtres (216) disposées autour de la tête de travail (202), une rotation d'un arbre (206) étant conçue pour créer un vortex (220, 222) afin de diriger un fluide devant les surfaces de percussion (224) et vers l'extérieur à travers les fenêtres (216).

19. Cathéter selon l'une quelconque des revendications précédentes, caractérisé en ce que l'ensemble d'entraînement (22) comporte un câble d'entraînement allongé (48) conçu pour tourner à l'intérieur d'un palier hélicoïdal (49) qui s'étend le long du cathéter.

20. Cathéter selon la revendication 20, caractérisé en ce que le palier hélicoïdal (49) est formé d'un fil métallique qui possède une configuration en section transversale sensiblement rectangulaire.

21. Cathéter selon la revendication 20 ou 21, caractérisé en ce que le câble (48) et le palier (49) présentent des surfaces de contact (48A, 49A) importantes, respectivement destinées à diminuer une usure.
